# EUROPÄISCHE PATENTANMELDUNG

(11) **EP 4 628 547 A1**
(43) Veröffentlichungstag der Anmeldung: **08.10.2025**
(21) Anmeldenummer: 25165035.4
(22) Anmeldetag: 20.03.2025
(51) Int. Cl.: C09B 57/12

(54) **SULFONYL-VERBRÜCKTE PERINONE**

(30) Priorität: 27.03.2024 EP 24166725
(71) Anmelder: LANXESS Deutschland GmbH, 50569 Köln (DE)
(72) Erfinder: MAY, Lars, 40764 Langenfeld (DE); KISCHKEWITZ, Marvin, 40217 Düsseldorf (DE); Schmauser, Sabine, 51371 Leverkusen (DE)

(57) **Zusammenfassung**

Die Erfindung betrifft Sulfonyl-verbrückte Perinone in Form von Verbindungen der allgemeinen Formel (I) oder deren isomere Formen 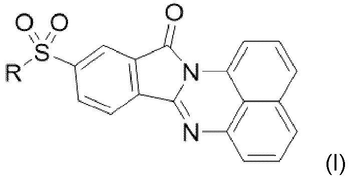 worin
R
für C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht,
ein Verfahren zu ihrer Herstellung sowie ihre Verwendung zum Massefärben von Kunststoffen.

## Beschreibung

Die Erfindung betrifft Sulfonyl-verbrückte Perinone und ihre isomeren Formen, ein Verfahren zu ihrer Herstellung, sowie ihre Verwendung in Kunststoff basierten Spritzgusserzeugnissen, insbesondere in Spritzgusserzeugnissen für die Nahrungsmittelindustrie.

Im Spritzguss zu verarbeitende Kunststoffe haben breiten Einsatz auch in der Nahrungsmittelindustrie gefunden. So lehrt EP 0 366 382 A1 den Einsatz von Polyamid zur Herstellung von Nahrungsmittel- und Getränkedosen.

WO 2019/069292 A1 beschreibt den Einsatz von Polyethylenterephthalat (PET) zur Herstellung von Getränkeflaschen durch Blasformen, insbesonder Spritzblasen oder Streckblasen. Prinzipiell lassen sich Polyester wie PET aber auch durch Spritzgießen, Extrusion oder Folienblasen verarbeiten. Mit Ultradur^{®} B1520 FC R01 (FC = Food Contact) bietet die BASF SE ein speziell für Verpackungszwecke von Nahrungsmitteln entwickeltes Polybutylenterephthalat mit hoher Fließfähigkeit an, das sich durch seine Steifigkeit, Festigkeit, Temperatur- und Chemikalienbeständigkeit auszeichnet.

Kunststoff basierte Gegenstände mit Lebensmittelkontakt können aber entweder aus ästhetischen Gründen, oder aber auch zur Unterscheidung ihres Inhalts, mit Farbstoffen eingefärbt sein. In jüngster Zeit sind jedoch in sensiblen Anwendungen wie dem Lebensmittelkontakt bisher gängige Farbstoffe nicht mehr gewünscht da diese in Verdacht stehen, ein gesundheitliches Risiko darzustellen; C. Barciela et. al., Food and Chemical Toxicology Volume 178, August 2023, 113935, Seiten 1 -12**.**

Aus DE 43 27 855 A1 sind u.A. gegebenenfalls substituierte Benzolsulfonyl-verbrückte Phthaloperinonfarbstoffe auf Basis von gegebenenfalls substituiertem 1,8-Diaminonaphthalin zum Massefärben von Kunststoffen bekannt. Durch Lösen dieser Phthaloperinonfarbstoffe in monomeren Komponenten eines Kunststoffs und inniges Vermischen werden transparente bzw. gedeckte brillante, orange bis violette Färbungen mit guter Hitzebeständigkeit sowie guter Licht- und Wetterechtheit erhalten. DE 43 27 855 A1 macht keine Aussage zur Verwendung solcher Phthaloperinonfarbstoffe in Kunststoff basierten Spritzgusserzeugnissen, insbesondere solchen für die Nahrungsmittelindustrie. Die gemäß DE 43 27 855 A1 Benzolsulfonyl-verbrückten Phthaloperinonfarbstoffe sind nicht rot!

Aufgabe der vorliegenden Erfindung war deshalb die Bereitstellung neuer, roter Farbstoffe die einerseits eine hohe Temperaturstabilität aufweisen um zusammen mit einem Kunststoff im Spritzgussverfahren verarbeitet zu werden, gleichzeitig aber ein geringeres Migrationsverhalten gegenüber üblicherweise in Kunststoffen mit Lebensmittelkontakt einzusetzenden Farbstoffen aufweisen.

Lösung der Aufgabe und Gegenstand der vorliegenden Erfindung sind Sulfonyl-verbrückte Perinone der allgemeinen Formel (I) oder ihre Isomere 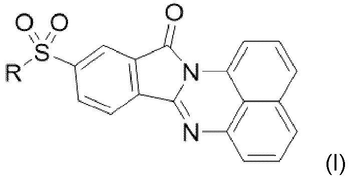 worin
R für C₁-C₆-Alkyl, Aryl, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht.

Insbesondere ist die Lösung der Aufgabe und Gegenstand der vorliegenden Erfindung gegebenenfalls substituierte 10,10'-, 9,9'- oder 9,10-Isomere des Sulfonyl-bis-12H-phthaloperin-12-ons.

Abgesehen von ihrer roten Farbe zeigen Sulfonyl-verbrückte Perinone der Formel (I) und ihre isomeren Formen, insbesondere gegebenenfalls substituierte 10,10'-, 9,9'- oder 9,10-Isomere des Sulfonyl-bis-12H-phthaloperin-12-ons, überraschenderweise besonders hohe Temperaturstabilitäten, womit sie insbesondere für den Einsatz im Kunststoffspritzguss geeignet sind und zeichnen sich zudem durch ein besonders niedriges Migrationsverhalten im bzw. aus dem Kunststoff im Vergleich zu den im Lebensmittelbereich gemäß C. Barciela et. al., Food and Chemical Toxicology Volume 178, August 2023, 113935, Seiten 1 -12 üblicherweise eingesetzten Farbstoffen aus. Temperaturstabilität ist eine wesentliche Voraussetzung für Farbstoffe, die in mittels Spritzgießen zu verarbeitenden Kunststoffen eingesetzt werden. Beim Spritzgießen wird eine Kunststoffschmelze unter hohem Druck, zumeist im Bereich von 500 bis 2000 bar und bei Temperaturen im Bereich von 200°C bis 300°C durch die geöffnete Düse und den Anguss bzw. das Angusssystem eines Spritzgießwerkzeugs in einen formgebenden Hohlraum gedrückt werden; siehe:
**https://www.kunststoffe.de/a/grundlagenartikel/spritzgiessen-254055.**

Zur Klarstellung sei angemerkt, dass vom Rahmen der vorliegenden Erfindung alle nachfolgend aufgeführten allgemeinen oder in Vorzugsbereichen genannten Definitionen und Parameter in beliebigen Kombinationen umfasst sind. Dies betrifft ebenso die Kombination von Mengenangaben einzelner Komponenten in Bezug auf erfindungsgemäß beanspruchte Verfahren und Verwendungen. Sofern nicht anders angegeben beziehen sich die im Rahmen dieser Anmeldung genannten Normen auf die zum Anmeldetag dieser Erfindung geltende Fassung. Sofern nicht anders angegeben handelt es sich bei Prozentangaben um Gewichtsprozente. Die peri-Stellung entspricht eigentlich der 1,8-Stellung im Naphthalin. Diese Bedeutung der peri-Stellung wird sowohl in der Literatur, als auch im Sinne der vorliegenden Anmeldung auf Arylene angewandt, die mehr als zwei miteinander kondensierte Benzolringe aufweisen.

Aryl oder eine Arylgruppe, abgekürzt Ar, ist ein organisch-chemischer Rest mit einem aromatischen Grundgerüst. Aryl ist somit die allgemeine Bezeichnung für eine einwertige Atomgruppe, die sich von aromatischen Kohlenwasserstoffen durch Entzug eines an den Ring gebundenen Wasserstoffatoms ableiten. Die meisten Arylreste leiten sich vom Benzol (C₆H₆) ab, die einfachste Arylgruppe und erfindungsgemäß bevorzugt ist die Phenylgruppe (Ph), (-C₆H₅).

Alkyl oder eine Alkylgruppe ist ein Teil eines Moleküls, der aus miteinander verbundenen Kohlenstoff- und Wasserstoffatomen besteht. Die einfachste Alkylgruppe ist die Methylgruppe -CH₃. Weitere erfindungsgemäß bevorzugte Alkylgruppen sind die Ethylgruppe -CH₂-CH₃ oder die n-Propylgruppe -CH₂-CH₂-CH₃. Die allgemeine Formel für Alkylgruppen in Form einer Kette ist CₙH₂ₙ₊₁.

Acyl oder eine **Acylgruppe** bezeichnet eine funktionelle Gruppe mit der allgemeinen Struktur R-(C=O)-, wobei R ein Organyl-Rest, vorzugsweise Alkyl-, Aryl- oder eine heteroaromatische Gruppe, oder ein Wasserstoffatom ist. Die Acylgruppe findet sich in Verbindungen wie Aldehyden, oder Carbonsäuren, oder Carbonsäurederivaten, insbesondere Carbonsäurechloriden, in denen formal betrachtet eine OH-Gruppe, ein Wasserstoffatom bzw. ein Chloratom durch einen Rest ersetzt wurde.

Zur Klarstellung sei ferner angemerkt, dass die Formel (I) nur eines von mehreren möglichen Isomeren wiedergibt.Formel (I) nur eines von mehreren möglichen Isomeren wiedergibt, wobei man als Isomere gemäß **https://de.wikipedia.org./wiki/Isomerie** das Auftreten von zwei oder mehreren chemischen Verbindungen mit gleicher Summenformel und Molekülmasse bezeichnet, die sich jedoch in der Verknüpfung oder der räumlichen Anordnung der Atome unterscheiden und sich durch unterschiedliche Strukturformeln darstellen lassen. Im Falle der erfindungsgemäßen Sulfonyl-verbrückten Perinone der Formel (I) handelt es sich um Stellungsisomere bei denen die gleiche funktionelle Gruppe an verschiedenen Positionen lokalisiert sein kann, im vorliegenden Fall an Position 9 oder an Position 10 der Perinon-Struktur. Zur Klarstellung sei ferner angemerkt, dass die erfindungsgemäß insbesondere bevorzugten, gegebenenfalls substituierten Sulfonyl-bis-12H-phthaloperin-12-one in Form ihrer 10,10'-, 9,9'- oder 9,10-Isomere vorliegen.

Gegebenenfalls substituiert bzw. unsubstituiert bezieht sich im Rahmen der vorliegenden Erfindung auf die durch das im erfindungsgemäßen Verfahren einzusetzende gegebenenfalls substituierte oder unsubstituierte 1,8-Naphthalendiamin bzw. den Naphthylrest bzw. auf die daraus im erfindungsgemäßen Sulfonyl-verbrückten Perinon der Formel (I) bzw. im insbesondere bevorzugten Sulfonyl-bis-12H-phthaloperin-12-on vorliegenden beiden Naphthylreste. Vorzugsweise können die beiden Naphthylreste ein bis maximal zwölf gleiche oder verschiedene Substituenten aufweisen. In diesem Fall weist das im erfindungsgemäßen Verfahren einzusetzende 1,8-Naphthalendiamin zusätzlich zu den für die Umsetzung mit 5,5-Sulfonylbis(isobenzofuran-1,3-dion) erforderlichen Aminogruppen wenigstens einen weiteren Substituenten auf. Vorzugsweise ist hierfür wenigstens ein Substituent aus der Reihe Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl auszuwählen.**Bevorzugte Ausführungen der Erfindung**

Erfindungsgemäß bevorzugte Isomere bzw. isomere Formen der 10,10'-Sulfonylverbückten Perinone sind die 9,9'-Sulfonyl-verbrückten Perinone, die 10,9-Sulfonyl-verbrückten Perinone und die 9,10-Sulfonyl-verbrückten Perinone.

Erfindungsgemäße Isomere bzw. isomere Formen der insbesondere bevorzugten gegenenenfalls substituierten Sulfonyl-bis-12H-phthaloperin-12-one sind das gegebenenfalls substituierte 10,10'-Sulfonyl-bis-12H-phthaloperin-12-on, das gegebenenfalls substituierte 9,10-Sulfonyl-bis-12H-phthaloperin-12-on und das gegebenenfalls substituierte 9,9-Sulfonyl-bis-12H-phthaloperin-12-on.

Vorzugsweise betrifft die Erfindung unsubstituiertes 10,10'-Sulfonyl-bis-12H-phthaloperin-12-on gemäß Formel (II), unsubstituiertes 9,10-Sulfonyl-bis-12H-phthaloperin-12-on gemäß Formel (III) und unsubstituiertes 9,9-Sulfonyl-bis-12H-phthaloperin-12-on gemäß Formel (IV).

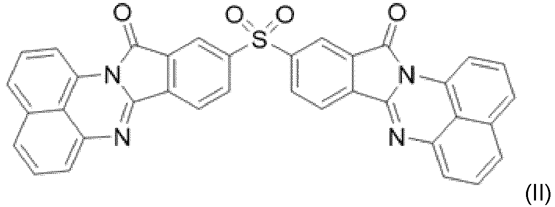

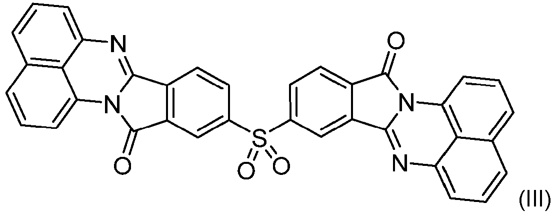

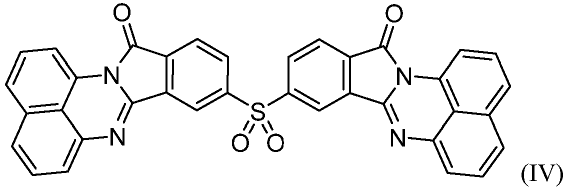

Erfindungsgemäß insbesondere bevorzugte substituierte Isomere von Sulfonyl-bis-12H-phthaloperin-12-on gemäß der Formeln (II), (III) und (IV) zeichnen sich durch wenigstens einen Substutuenten an wenigstens einem der beiden Naphthylreste aus. Vorzugsweise können die beiden Naphthylreste ein bis maximal zwölf gleiche oder verschiedene Substituenten aufweisen. Bevorzugte Substituenten sind auszuwählen aus der Reihe Chlor, Brom, Nitro, Methoxy, NH2, Benzyloxy, Hydroxy, -SO₂O(C₆H_{5\}), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl.

Bevorzugte Kunststoffe im Sinne der vorliegenden Erfindung sind solche, wie sie im Spritzguss, in der Extrusion oder beim Blasformen eingesetzt werden, insbesondere im Spritzguss. Vorzugsweise handelt es sich um Thermoplaste, besonders bevorzugt Vinylpolymere, Polyester, Polyolefine oder Polyamide. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Vorzugsweise betrifft die Erfindung Sulfonyl-verbrückte Perinone der allgemeinen Formel (I) oder ihre Isomere 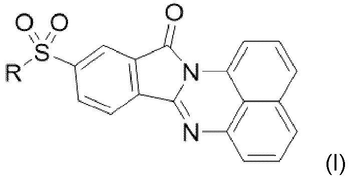 worin R für C₁-C₆-Alkyl, Aryl, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht mit einem Farbabstand ΔE <20 von den L*a*b* Koordinaten zu einer mit "3" beginnenden Farbnummer der RAL-Farbtabelle.

Besonders bevorzugt betrifft die Erfindung Sulfonyl-verbrückte Perinone der Formel (I), oder ihre Isomere worin R für 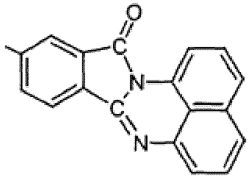 steht.

In einer alternativen oder bevorzugten Ausführungsform betrifft die vorliegende Erfindung Sulfonyl-verbrückte Perinone der Formel (I), worin wenigstens einer der Phenylringe in der Perinon Struktur wenigstens einen Substituenten der Reihe Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl aufweist.

Vorzugsweise verbrückt die Sulfonylgruppe zwei derselben Perinonstrukturen. Erfindungsgemäß ganz besonders bevorzugt werden durch die Sulfonylgruppe zwei 12H-Phthaloperin-12-on-Reste miteinander verbrückt, wobei das 12H-Phthaloperin-12-on selbst bereits als Farbstoff Solvent Orange 60 [CAS No. 6925-69-5] aus EP 1 245 645 A1 bekannt ist. Seine Synthese wird in EP 780 444 A2**,** Beispiel 1 beschrieben. Das Resultat der erfindungsgemäßen Sulfonyl-Verbrückung zweier 12H-Phthaloperin-12-on-Reste ist der erfindungsgemäß ganz besonders bevorzugte Sulfonyl-verbrückte Perinonfarbstoff der Formel (II) mit der Summenformel C₃₆H₁₈N₄O₄S bzw. 10,10'-Sulfonyl-bis-12H-phthaloperin-12-on und seine Isomere.

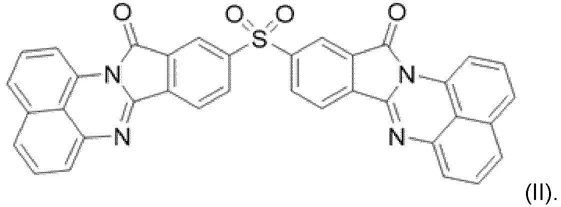

Erfindungsgemäß bevorzugte Isomere des 10,10'-Sulfonyl-bis-12H-phthaloperin-12-on sind das 9,9'-Sulfonyl-bis-12H-phthaloperin-12-on, das 10,9-Sulfonyl-bis-12H-phthaloperin-12-on und das 9,10-Sulfonyl-bis-12H-phthaloperin-12-on.

### Tab.1 RAL-Farbtabelle

Im Rahmen der vorliegenden Erfindung gilt als Rot eine Farbe, die im RAL Farbsystem nach **https://de.wikipedia.org/wiki/RAL-Farbe#Rot** in der RAL Farbtabelle eine Farbnummer hat, die mit einer "3" beginnt. Im Einzelnen unterscheidet man zum Anmeldetag der vorliegenden Erfindung Rottöne wie folgt:

| | | L* | a* | b* |
|---|---|---|---|---|
| RAL 3000 | Feuerrot | 44 | 50 | 39 |
| RAL 3001 | Signalrot | 41 | 49 | 33 |
| RAL 3002 | Karminrot | 41 | 49 | 35 |
| RAL 3003 | Rubinrot | 36 | 47 | 27 |
| RAL 3004 | Purpurrot | 31 | 38 | 18 |
| RAL 3005 | Weinrot | 26 | 33 | 15 |
| RAL 3007 | Schwarzrot | 23 | 17 | 7 |
| RAL 3009 | Oxidrot | 29,27 | 24,59 | 16,51 |
| RAL 3011 | Braunrot | 34,52 | 28,66 | 13,44 |
| RAL 3012 | Beigerot | 63,81 | 20,79 | 20,45 |
| RAL 3013 | Tomatenrot | 40,70 | 36,67 | 21,37 |
| RAL 3014 | Altrosa | 60,17 | 32,49 | 12,58 |
| RAL 3015 | Hellrosa | 71,23 | 21,59 | 4,98 |
| RAL 3016 | Korallenrot | 44,70 | 37,92 | 23,96 |
| RAL 3017 | Rosé | 54,24 | 44,26 | 16,87 |
| RAL 3018 | Erdbeerrot | 50,77 | 49,15 | 19,86 |
| RAL 3020 | Verkehrsrot | 46 | 59 | 54 |
| RAL 3022 | Lachsrot | 56,06 | 38,90 | 29,70 |
| RAL 3024 | Leuchtrot | 51,32 | 82,52 | 71,62 |
| RAL 3026 | Leuchthellrot | 59 | 70 | 59 |
| RAL 3027 | Himbeerrot | 43,07 | 46,96 | 15,81 |
| RAL 3028 | Reinrot | 51 | 58 | 46 |
| RAL 3031 | Orientrot | 46 | 45 | 25 |
| RAL 3032 | Perlrubinrot | 26,88 | 41,34 | 19,40 |
| RAL 3033 | Perlrosa | 44,29 | 45,11 | 28,62 |

Dargestellt sind in hier die für Rot geräteunabhängigen CIE L*a*b* Farbwerte für den jeweiligen RAL Wert: L* steht für die Luminanz, a* beschreibt den Farbort bezüglich der Rot-Grün-Achse und b* beschriebt den Farbort bezüglich der Gelb-Blau-Achse unter Verwendung von D65 Normlicht mit einem 10° Sichtfeld eines Normalbeobachters. Das Farbmodell ist in der **EN ISO 11664-4** "Colorimetry - Part 4: CIE 1976 L*a*b* Colour space" genormt. Zu L*a*b*-Farbraum (auch: CIELAB) siehe: **https://de.wikipedia.org/wiki/Lab-Farbraum.** Jede Farbe im Farbraum ist durch einen Farbort mit den kartesischen Koordinaten {L*, a*, b*} definiert. Die a*b*-Koordinatenebene wurde in Anwendung der Gegenfarbentheorie konstruiert. Auf der a*-Achse liegen sich Grün und Rot gegenüber, die b*-Achse verläuft zwischen Blau und Gelb. Komplementäre Farbtöne stehen sich jeweils um 180° gegenüber, in ihrer Mitte (dem Koordinatenursprung a*=0, b*=0) liegen alle unbunten Farben.

Die L*-Achse beschreibt die Helligkeit (Luminanz) der Farbe mit Werten von 0 bis 100. In der Darstellung steht diese im Nullpunkt senkrecht auf der a*b*-Ebene. Sie kann auch als Neutralgrauachse bezeichnet werden, denn zwischen den Endpunkten Schwarz (L*=0) und Weiß (L*=100) sind alle unbunten Farben (Grautöne) enthalten. Die a*-Achse beschreibt den Grün- oder Rotanteil einer Farbe, wobei negative Werte für Grün und positive Werte für Rot stehen. Die b*-Achse beschreibt den Blau- oder Gelbanteil einer Farbe, wobei negative Werte für Blau und positive Werte für Gelb stehen.

Die a*-Werte reichen von ca. -170 bis +100, die b*-Werte von -100 bis +150, wobei die Maximalwerte nur bei mittlerer Helligkeit bestimmter Farbtöne erreicht werden. Der CIELAB-Farbkörper hat im mittleren Helligkeitsbereich seine größte Ausdehnung, die aber je nach Farbbereich unterschiedlich in Höhe und Größe ist.

Erfindungsgemäß umfasst sind aber auch zu Rot ähnliche Farbtöne die einen Farbabstand ΔE <20 von den L*a*b* Koordinaten zu einer mit "3" beginnenden Farbnummer der RAL-Farbtabelle für die Farbe Rot aufweisen.

Erfindungsgemäß bevorzugt sind rote Sulfonyl-verbrückte Perinone der allgemeinen Formel (I) oder ihre Isomere die einen Farbabstand ΔE <20 von den L*a*b* Koordinaten zu einer mit "3" beginnenden Farbnummer der RAL-Farbtabelle für die Farbe Rot aufweisen mit einem Gelbstich wobei a* > 0 und b* ≥ 25 gilt. Damit gilt für den Bunttonwinkel h: h°ₘᵢₙᵢₘₐₗ = 14° und h°ₘₐₓᵢₘₐₗ < 90°, wobei alle Farborte, die dazwischen liegen, mit umfasst sind.

Insbesondere bevorzugt sind **gegebenenfalls substituierte** 10,10'-, 9,9'- oder 9,10-Isomere von Sulfonyl-bis-12H-phthaloperin-12-on mit einem Farbabstand ΔE <20 von den L*a*b* Koordinaten zu einer mit "3" beginnenden Farbnummer der RAL-Farbtabelle gemäß Farbmodell in der **EN ISO 11664-4.** Die mit "3" beginnenden Farbnummern der RAL-Farbtabelle gemäß Farbmodell in der **EN ISO 11664-4** stehen für die Farbe Rot.

Insbesondere besonders bevorzugt sind **gegebenenfalls substituierte** 10,10'-, 9,9'- oder 9,10-Isomere von Sulfonyl-bis-12H-phthaloperin-12-on die einen Farbabstand ΔE <20 von den L*a*b* Koordinaten zu einer mit "3" beginnenden Farbnummer der RAL-Farbtabelle gemäß Farbmodell in der **EN ISO 11664-4** aufweisen mit einem Gelbstich der mit a* > 0 und b* ≥ 25 definiert ist, womit für den Bunttonwinkel h: h°ₘᵢₙᵢₘₐₗ = 14° und h°ₘₐₓᵢₘₐₗ < 90° gilt, wobei alle Farborte, die dazwischen liegen, mit umfasst sind.

Insbesondere bevorzugt sind aber auch **unsubstituierte** 10,10'-, 9,9'- oder 9,10-Isomere von Sulfonyl-bis-12H-phthaloperin-12-on mit einem Farbabstand ΔE <20 von den L*a*b* Koordinaten zu einer mit "3" beginnenden Farbnummer der RAL-Farbtabelle gemäß Farbmodell in der **EN ISO 11664-4.** Die mit "3" beginnenden Farbnummern der RAL-Farbtabelle gemäß Farbmodell in der **EN ISO 11664-4** stehen für die Farbe Rot.

Insbesondere besonders bevorzugt sind schließlich **unsubstituierte** 10,10'-, 9,9'- oder 9,10-Isomere von Sulfonyl-bis-12H-phthaloperin-12-on die einen Farbabstand ΔE <20 von den L*a*b* Koordinaten zu einer mit "3" beginnenden Farbnummer der RAL-Farbtabelle gemäß Farbmodell in der **EN ISO 11664-4** aufweisen mit einem Gelbstich der mit a* > 0 und b* ≥ 25 definiert ist, womit für den Bunttonwinkel h: h°ₘᵢₙᵢₘₐₗ = 14° und h°ₘₐₓᵢₘₐₗ < 90° gilt, wobei alle Farborte, die dazwischen liegen, mit umfasst sind.**Verfahren**

Ein möglicher Syntheseweg zur Herstellung Sulfonyl-verbrückter Perinone der Formel (I) und ihrer isomeren Formen ist die Umsetzung oder Kondensation Sulfonyl-substituierter Isobenzofurandione mit wenigstens einem aromatischen Diamin der Reihe 1,8-Naphthylendiamin, Chlor-1,8-naphthylendiamin, Dichlor-1,8-naphthylendiamin, Methyl-1,8-naphthylendiamin, Dimethyl-1,8-naphthylendiamin, Methoxy-1,8-naphthylendiamin, Ethoxy-1,8-naphthylendiamin, Acetamino-1,8-naphthylen-diamin und 1,8-Diaminoacetnaphthylen. Besonders bevorzugt ist 1,8-Naphthylendiamin. Dieser Syntheseweg ist besonders bevorzugt zur Herstellung des erfindungsgemäß insbesondere bevorzugten unsubstituierten Sulfonyl-verbrückten Perinons der Formel (II) und seiner isomeren Formen anzuwenden, wofür 5,5-Sulfonylbis(isobenzofuran-1,3-dion) mit 1,8-Naphthalendiamin umgesetzt wird.

Vorzugsweise werden pro Mol 5,5-Sulfonyl-verbrücktem Isobenzofurandion wenigstens 2 Mol gegebenenfalls substituiertes aromatisches Diamin eingesetzt. In einigen Ausführungsformen können auch mehr als 2 Mol des gegebenenfalls substituierten aromatischen Diamins eigesetzt werden, wobei allerdings der stöchiometrische Einsatz der Reaktanden bevorzugt ist.

Da zur Synthese erfindungsegmäßer Sulfonyl-bis-12H-phthaloperin-12-one pro Mol Sulfonyl-substituiertem Isobenzofurandion wenigstens 2 Mol gegebenenfalls substituiertem aromatischen Diamins eingesetzt werden, können auch Sulfonyl-bis-12H-phthaloperin-12-one hergestellt werden, worin die beiden 1,8-Naphtyhlreste unterschiedlich substituiert sind. Unterschiedlich substituiert bedeutet in diesem Fall an unterschiedlichen Positionen des jeweiligen Naphthylrests und/oder unterschiedliche Substituenten am jeweiligen Naphthylrest aus der Reihe Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl.Die Kondensation der Reaktanden kann dabei direkt durch Zusammenschmelzen entsprechender Mol-Äquivalente der Reaktanden bei einer Temperatur im Bereich von 120°C bis 250°C erfolgen.

Vorzugsweise erfolgt die Umsetzung der Reaktanden in einem Lösungsmittel bei einer Temperatur im Bereich von 110°C bis 220°C, gegebenenfalls unter Druck, wobei eine destillative Entfernung des Reaktionswassers erfolgen kann. Vorzugsweise wird für die Kondensation wenigstens ein Lösungsmittel eingesetzt, ausgewählt aus der Gruppe Chlorbenzol, o-Dichlorbenzol, Trichlorbenzol, Xylol, Dimethylformamid, N-Methylpyrrolidon, Eisessig, Propionsäure, Phenol, Kresole, Phenoxyethanol, Glykole und deren Mono- und Dialkylether, Alkohole, insbesondere Methanol, Ethanol, i-Propanol, Wasser und wäßrige Lösungsmittel, insbesondere verdünnte Schwefelsäure. Erfindungsgemäß besonders bevorzugt wird Phenol eingesetzt.

In einer Ausführungsform kann die Reaktion unter Zusatz wenigstens eines sauren Katalysators erfolgen. Vorzugsweise werden in diesem Fall Katalysatoren eingesetzt, ausgewählt aus der Gruppe Zinkchlorid, p-Toluolsulfonsäure, Salzsäure, Schwefelsäure, organische Säuren.

Die Aufarbeitung eines Reaktionsansatzes zur Herstellung der Sulfonyl-verbrückten Perinone der Formel (I) und ihrer isomeren Formen, insbesondere zur Herstellung gegebenenfalls substituierter Sulfonyl-bis-12H-phthaloperin-12-one in Form ihrer 10,10'-, 9,9'- oder 9,10-Isomere, erfolgt vorzugsweise durch Verdünnen mit Alkoholen, vorzugsweise Methanol, Ethanol, Propanol oder Butanol. Auch aromatische Verdünnungsmittel, vorzugsweise Chlorbenzol oder Toluol, können verwendet werden. Das erfindungsgemäße Verfahren liefert die Sulfonyl-verbrückten Perinone der Formel (I) und ihre isomeren Formen vorzugsweise in Ausbeuten von 85 bis 95 % der Theorie.

Die erfindungsgemäßen Sulfonyl-verbrückten Perinone der Formel (I) und ihre isomeren Formen, insbesondere die gegebenenfalls substituierten Sulfonyl-bis-12H-phthaloperin-12-one in Form ihrer 10,10'-, 9,9'- oder 9,10-Isomere, eignen sich hervorragend zum Massefärben von Kunststoffen. Unter Massefärben werden hierbei insbesondere Verfahren verstanden, bei denen der Farbstoff in die geschmolzene Kunststoffmasse eingearbeitet wird, vorzugsweise unter Zuhilfenahme eines Extruders, oder indem der Farbstoff bereits Edukten zur Herstellung eines jeweiligen Kunststoffs, vorzugsweise den jeweiligen Monomeren vor der Polymerisation, zugesetzt wird.

Besonders bevorzugte, mittels erfindungsgemäßer Sulfonyl-verbrückter Perinone der Formel (I) und ihrer isomeren Formen, insbesondere mittels gegebennfalls substituierter Sulfonyl-bis-12H-phthaloperin-12-one in Form ihrer 10,10'-, 9,9'- oder 9,10-Isomere, zu färbende Kunststoffe sind im Spritzguss einzusetzende Thermoplaste, vorzugsweise Vinylpolymere, Polyester, Polyolefine oder Polyamide, insbesondere Kunststoffe für das Spritzgießen. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Ganz besonders bevorzugte, mittels erfindungsgemäßer Sulfonyl-verbrückter Perinone der Formel (I) und entsprechender Isomere, insbesondere mittels gegebenenfalls substituierter Sulfonyl-bis-12H-phthaloperin-12-one in Form ihrer 10,10'-, 9,9'- oder 9,10-Isomere, zu färbende Kunststoffe sind Polystyrol, Styrol-Acrylnitril-Copolymere, Styrol-Butadien-Copolymere, Styrol-Butadien-Acrylnitril-Terpolymere, Polymethacrylat, Polyethylenterephthalat, Polyamid 6 oder Polyamid 66.

Die erfindungsgemäß als Farbstoffe einzusetzenden Sulfonyl-verbrückten Perinone der Formel (I) und entsprechende Isomere, insbesondere die gegebenenfalls substituierten Sulfonyl-bis-12H-phthaloperin-12-one in Form ihrer 10,10'-, 9,9'- oder 9,10-Isomere, werden vorzugsweise in feinverteilter Form zur Anwendung gebracht, gegebenenfalls unter Einsatz zusätzlich von wenigstens einem Dispergiermittel.

Werden die erfindungsgemäß als Farbstoffe einzusetzenden Sulfonyl-verbrückten Perinone der Formel (I) und entsprechende Isomere, insbesondere die gegebenenfalls substituierten Sulfonyl-bis-12H-phthaloperin-12-one in Form ihrer 10,10'-, 9,9'- oder 9,10-Isomere, nach der Polymerisation des jeweiligen Kunststoffs eingesetzt, so werden sie mit dem Kunststoffgranulat trocken vermischt oder vermahlen und dieses Gemisch vorzugsweise auf Mischwalzen oder in Schnecken plastifiziert und homogenisiert. Man kann die erfindungsgemäß als Farbstoffe einzusetzenden Sulfonyl-verbrückten Perinone der Formel (I) und ihre isomeren Formen, insbesondere die gegebenenfalls substituierten Sulfonyl-bis-12H-phthaloperin-12-one in Form ihrer 10,10'-, 9,9'- oder 9,10-Isomere, aber auch der schmelzflüssigen Masse eines Kunststoffs zugeben und darin durch Rühren homogen verteilen. Ein derart vorgefärbter Kunststoff wird dann üblicherweise bzw. vorzugsweise durch Verspinnen zu Borsten, Fäden oder durch Extrusion, Blasformen oder im Spritzguß zu Formteilen weiterverarbeitet.

Da die erfindungsgemäß als Farbstoffe einzusetzenden Sulfonyl-verbrückten Perinone der Formel (I) und entsprechende Isomere, insbesondere die gegebenenfalls substituierten Sulfonyl-bis-12H-phthaloperin-12-one in Form ihrer 10,10'-, 9,9'- oder 9,10-Isomere, gegenüber Polymerisationskatalysatoren, insbesondere gegenüber Peroxiden, beständig sind, ist es auch möglich, diese den monomeren Edukten der jeweiligen Kunststoffe zuzusetzen und die eingefärbten monomeren Edukte dann in Gegenwart von Polymerisationskatalysatoren zu polymerisieren. Dazu werden die erfindungsgemäßen Sulfonyl-verbrückten Perinone der Formel (I) und ihre isomeren Formen, insbesondere die gegebenenfalls substituierten Sulfonyl-bis-12H-phthaloperin-12-one in Form ihrer 10,10'-, 9,9'- oder 9,10-Isomere, vorzugsweise in den monomeren Edukten eines jeweiligen Kunststoffs gelöst oder mit ihnen innig vermischt. Vorzugsweise werden die erfindungsgemäßen Sulfonyl-verbrückten Perinone der Formel (I) und ihre isomeren Formen, insbesondere die gegebenenfalls substituierten Sulfonyl-bis-12H-phthaloperin-12-one in Form ihrer 10,10'-, 9,9'- oder 9,10-Isomere, zum Färben der genannten Kunststoffe bzw. Polymere in Mengen im Bereich von 0,0001 bis 1 Gew.-%, insbesondere im Bereich von 0,01 bis 0,5 Gew.-%, jeweils bezogen auf die Kunststoff- bzw. Polymermenge, eingesetzt.

Durch Zusatz von im jeweiligen Kunststoff bzw. in den Polymeren unlöslichen Pigmenten, insbesondere Titandioxid, können entsprechende wertvolle gedeckte Färbungen erhalten werden. Diese unlöslichen Pigmente, insbesondere Titandioxid, werden vorzugsweise in Mengen im Bereich von 0,01 bis 10 Gew.-%, vorzugsweise im Bereich von 0,1 bis 5 Gew.-%, bezogen auf die Polymermenge, eingesetzt.

Erfindungsgemäß können auch Mischungen verschiedener erfindungsgemäßer Sulfonyl-verbrückter Perinone und/oder Mischungen erfindungsgemäßer Sulfonyl-verbrückter Perinone der Formel (I) sowie ihrer isomeren Formen, insbesondere Mischungen der gegebenenfalls substituierten Sulfonyl-bis-12H-phthaloperin-12-one in Form ihrer 10,10'-, 9,9'- oder 9,10-Isomere, mit anderen Farbstoffen und/oder anorganischen bzw. organischen Pigmenten eingesetzt werden.

### Weitere Verwendungen

Die vorliegende Erfindung betrifft die Verwendung Sulfonyl-verbrückter Perinone der Formel (I) und entsprechender Isomere, insbesondere die Verwendung gegebenenfalls substituierter Sulfonyl-bis-12H-phthaloperin-12-one in Form ihrer 10,10'-, 9,9'- oder 9,10-Isomere, zum Massefärben von Kunststoffen unter gleichzeitiger Reduzierung des nach **DIN 53775-3** zu bestimmenden Migrationsverhaltens in diesen Kunststoffen.

Vorzugsweise handelt es sich bei den Kunststoffen um solche, die im Spritzguss, bei der Extrusion oder beim Blasformen eingesetzt werden. Vorzugsweise handelt es sich bei den Kunststoffen um Vinylpolymere, Polyester, Polyolefine oder Polyamide, insbesondere bevorzugt um Polyamid 6 oder Polycarbonat.

Vorzugsweise handelt es sich um Kunststoffe für das Spritzgießen. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Bevorzugt betrifft die Verwendung Sulfonyl-verbrückte Perinone der Formel (I) und deren Isomere, worin R für einen Rest 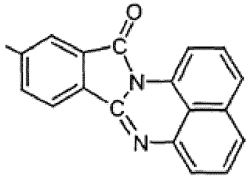 steht,
besonders bevorzugt ist die Verwendung von 10,10'-Sulfonyl-bis-12H-phthaloperin-12-on und seiner Isomere. Insbesondere bevorzugt sind in diesem Fall alle Naphthylreste unsubstituiert. Bevorzugt betrifft die Verwendung deshalb die unsubstituierten 10,10'-, 9,9'- oder 9,10-Isomere von Sulfonyl-bis-12H-phthaloperin-12-on. Die vorliegende Erfindung betrifft aber auch die Verwendung Sulfonyl-verbrückter Perinone der Formel (I) und entsprechender Isomere, insbesondere die Verwendung gegebenenfalls substituierter Sulfonyl-bis-12H-phthaloperin-12-one in Form ihrer 10,10'-, 9,9'- oder 9,10-Isomere, als Farbstoffe zum Massefärben von Kunststoffen bei gleichzeitiger Steigerung der nach **DIN EN 12877** zu bestimmenden Temperaturstabilität von Polymerzusammensetzungen auf Basis dieser Farbstoffe in diesen Kunststoffen. Vorzugsweise handelt es sich um Kunststoffe für das Spritzgießen, das Blasformen oder für die Extrusion, insbesondere für das Spritzgießen. Bevorzugte Kunststoffe sind Vinylpolymere, Polyester, Polyolefine oder Polyamide, insbesondere bevorzugt sind Polyamid 6 oder Polycarbonat. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Bevorzugt betrifft die Verwendung Sulfonyl-verbrückte Perinone der Formel (I) und deren Isomere worin R für einen Rest 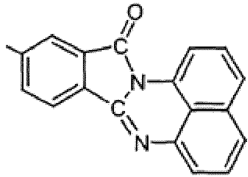 steht,
besonders bevorzugt ist 10,10'-Sulfonyl-bis-12H-phthaloperin-12-on und seine Isomere.

Die Erfindung betrifft bevorzugt die Verwendung gegebenenfalls substituierter 10,10'-, 9,9'- oder 9,10-Isomere von Sulfonyl-bis-12H-phthaloperin-12-on zum Massefärben von Kunststoffen zur Erzielung eines Farbabstands ΔE <20 von den L*a*b* Koordinaten zu einer mit "3" beginnenden Farbnummer der RAL-Farbtabelle gemäß Farbmodell in der **EN ISO 11664-4.** Die mit "3" beginnenden Farbnummern der RAL-Farbtabelle stehen für die Farbe Rot.

Bevorzugt ist die Verwendung gegebenenfalls substituierter 10,10'-, 9,9'- oder 9,10-Isomere von Sulfonyl-bis-12H-phthaloperin-12-on zum Massefärben von Kunststoffen zur Erzielung eines Farbabstands ΔE <20 von den L*a*b* Koordinaten zu einer mit "3" beginnenden Farbnummer der RAL-Farbtabelle gemäß Farbmodell in der **EN ISO 11664-4** mit einem Gelbstich der mit a* > 0 und b* ≥ 25 definiert ist, womit für den Bunttonwinkel h: h°ₘᵢₙᵢₘₐₗ = 14° und h°ₘₐₓᵢₘₐₗ < 90° gilt, wobei alle Farborte, die dazwischen liegen, mit umfasst sind.

Insbesondere bevorzugt ist die Verwendung gegebenenfalls substituierter 10,10'-, 9,9'- oder 9,10-Isomere von Sulfonyl-bis-12H-phthaloperin-12-on zum Massefärben von Kunststoffen zur Erzielung eines Farbabstands ΔE <20 von den L*a*b* Koordinaten zu einer mit "3" beginnenden Farbnummer der RAL-Farbtabelle gemäß Farbmodell in der **EN ISO 11664-4,** bei gleichzeitiger Steigerung der nach **DIN EN 12877** zu bestimmenden Temperaturstabilität von Polymerzusammensetzungen basierend auf diesen Isomeren des Sulfonyl-bis-12H-phthaloperin-12-on in Kunststoffen, wobei die im Rahmen der vorliegenden Erfindung beobachtete Steigerung der Temperaturstabilität für die untersuchten Kunststoffe im Bereich ihrer Verarbeitungsgrenzen in den untersuchten Polymerzusammensetzungen lag.

Vorzugsweise handelt es sich auch hier um Kunststoffe für das Spritzgießen, für das Blasformen oder für die Extrusion, insbesondere für das Spritzgießen. Vorzugsweise handelt es sich auch hier bei den Kunststoffen um Vinylpolymere, Polyester, Polyolefine oder Polyamide, insbesondere bevorzugt um Polyamid 6 oder Polycarbonat. Bevorzugte Polyolefine sind Polyethylen oder Polypropylen. Bevorzugte Vinylpolymere sind auch hier Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind auch hier Polyamid 6 oder Polyamid 66. Bevorzugt betrifft auch diese Verwendung unsubstituiertes 10,10'-Sulfonyl-bis-12H-phthaloperin-12-on und seine 9,9'- sowie 9,10-Isomere.

### Erzeugnisse

Die vorliegende Erfindung betrifft schließlich auch Extrusions-, Blasform- oder Spritzgusserzeugnisse basierend auf wenigstens einem Sulfonyl-verbrückten Perinon der allgemeinen Formel (I) oder seiner isomeren Formen 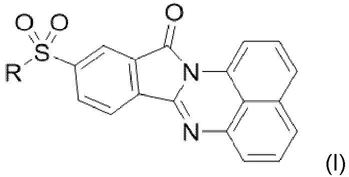 worin
- R: für C₁-C₆-Alkyl, Aryl, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht und
wenigstens einem Kunststoff, vorzugsweise wenigstens einem Thermoplasten, besonders bevorzugt wenigstens einem Kunststoff der Reihe Vinylpolymere, Polyester, Polyolefine und Polyamide. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Bevorzugt sind solche Extrusions-, Blasform- oder Spritzgusserzeugnisse, die auf Sulfonyl-verbrückten Perinonen der allgemeinen Formel (I) oder ihrer Isomere basieren, Ganz besonders bevorzugt sind Extrusions-, Blasform- oder Spritzgusserzeugnisse, die auf 10,10'-Sulfonyl-bis-12H-phthaloperin-12-on und seinen Isomeren basieren.

Die vorliegende Erfindung betrifft insbesondere Extrusions-, Blasform- oder Spritzgusserzeugnisse, vorzugsweise Extrusions-, Blasform- oder Spritzguss-Lebensmittelkontakterzeugnisse, basierend auf wenigstens einem gegebenenfalls substituierten Sulfonyl-bis-12H-phthaloperin-12-on in Form seiner 10,10'-, 9,9'- oder 9,10-Isomere und wenigstens einem Kunststoff, vorzugsweise wenigstens einem Thermoplasten, besonders bevorzugt wenigstens einem Kunststoff der Reihe Vinylpolymere, Polyester, Polyolefine und Polyamide. Bevorzugte Polyolefine sind Polyethylen oder Polypropylen. Bevorzugte Vinylpolymere sind Polystyrol, Styrol-Acrylnitril-Copolymere, Styrol-Butadien-Copolymere, Styrol-Butadien-Acrylnitril-Terpolymere, Polymethacrylat oder Polyvinylchlorid. Bevorzugte Polyester sind Polyethylenterephthalate, Polybutylenterephthalate, Polycarbonate oder Celluloseester. Bevorzugte Polyamide sind Polyamid 6 oder Polyamid 66.

Bevorzugt sind solche Extrusions-, Blasform- oder Spritzgusserzeugnisse, vorzugsweise Extrusions-, Blasform- oder Spritzguss-Lebensmittelkontakterzeugnisse, die auf unsubstituierten 10,10'-, 9,9'- oder 9,10-Isomeren von Sulfonyl-bis-12H-phthaloperin-12-on basieren.

Insbesondere besonders bevorzugt sind Extrusions-, Blasform- oder Spritzgusserzeugnisse, vorzugsweise Extrusions-, Blasform- oder Spritzguss-Lebensmittelkontakterzeugnisse, die auf unsubstituierten 10,10'-, 9,9'- oder 9,10-Isomeren von Sulfonyl-bis-12H-phthaloperin-12-on basieren und einen Farbabstand ΔE <20 von den L*a*b* Koordinaten zu einer mit "3" beginnenden Farbnummer der RAL-Farbtabelle gemäß Farbmodell in der **EN ISO 11664-4.** Ganz besonders bevorzugt sind solche sind Extrusions-, Blasform- oder Spritzgusserzeugnisse, die zudem einen Gelbstich aufweisen der mit a* > 0 und b* ≥ 25 definiert ist, womit für den Bunttonwinkel h: h°ₘᵢₙᵢₘₐₗ = 14° und h°ₘₐₓᵢₘₐₗ < 90° gilt, wobei alle Farborte, die dazwischen liegen, mit umfasst sind.

### Beispiele

### Beispiel 1

63 g Phenol wurde bei einer Temperatur von 80 °C aufgeschmolzen. In dieses wurden 5,27 g (32,7 mmol) 1,8-Diaminonaphthalin zugegeben und die Mischung für 30 Minuten gerührt. Anschließend wurden 6,32 g (16,8 mmol) 5,5-Sulfonylbis(isobenzofuran-1,3-dion) eingetragen. Diese Reaktionsmischung wurde auf 135 °C aufgeheizt, bei dieser Temperatur für 6 Stunden gehalten und das dabei entstehende Reaktionswasser abdestilliert. Die Reaktionsmischung wurde anschließend innerhalb von 1,5 h auf 100 °C abgekühlt und 65 g Methanol zugegeben. Es wurde 10 Stunden nachgerührt und die Reaktionsmischung auf 30 °C abgekühlt. Das Reaktionsprodukt wurde auf einer Nutsche isoliert und zunächst mit 40 g Methanol und dann mit 400 g Wasser gewaschen. Nach Trocknung in einem Vakuumtrockenschrank bei 80 °C und 150 mbar wurde ein Isomerengemisch mit im wesentlichen 10,10'-Sulfonyl-bis-12H-phthaloperin-12-on gemäß Formel (II) in einer Ausbeute von 99% mit roter Farbe nahe RAL 3028 erhalten.

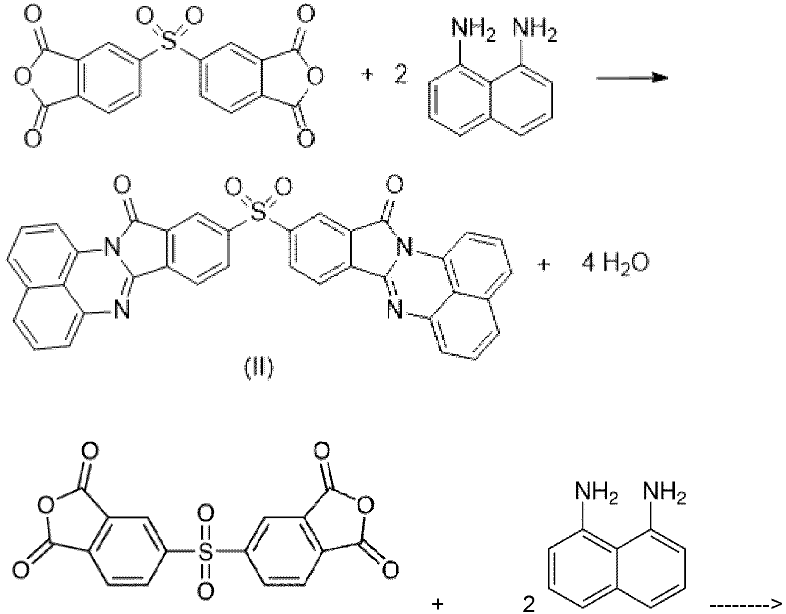

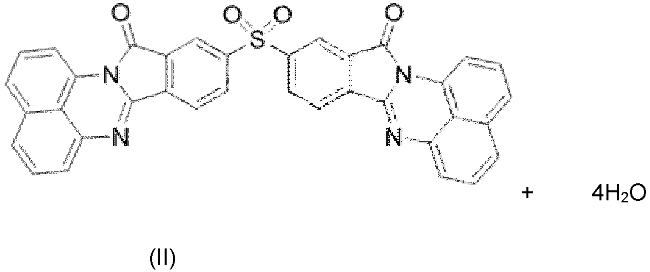

Die im Reaktionsschema dargestellte Formel (II) des Sulfonyl-verbrückten Farbstoffs 10,10'-Sulfonyl-bis-12H-phthaloperin-12-on gibt nur eine Form möglicher Isomere wieder, die im Rahmen der Synthese entstehen können.

Zum Nachweis der in der Beschreibung beschriebenen Verbesserungen der Eigenschaften wurden zunächst durch Compoundierung entsprechende Polymerzusammensetzungen aus Polycarbonat (Polymerzusammensetzung 1) und aus Polyamid 6 (Polymerzusammensetzung 2) mit 10,10'-Sulfonyl-bis-12H-phthaloperin-12-on und seinen Isomeren angefertigt. Die einzelnen Komponenten wurden hierzu in einem Zweiwellenextruder (Leistritz LSM 30-34der Leistritz AG (Nürnberg, Deutschland)) bei Temperaturen zwischen 240 und 260°C gemischt, als Strang ausgetragen, bis zur Granulierbarkeit abgekühlt und granuliert. Nach dem Trocknen (in der Regel zwei Tage bei 80°C im Vakuumtrockenschrank) erfolgte die Verarbeitung des Granulats im Spritzguss bei Temperaturen im Bereich von 240 bis 300°C zu Normprüfkörpern für die jeweiligen Prüfungen.

Im Rahmen der vorliegenden Erfindung wurde die Migration des 10,10'-Sulfonyl-bis-12H-phthaloperin-12-on und seiner Isomere anhand von Platten mit den Maßen 60•40•2 mm³ basierend auf den Thermoplasten Polycarbonat und Polyamid 6 gemäß der Norm **DIN 53775-3** geprüft. Dabei wurde der mit dem Sulfonyl-verbrückten Perinon mit der Summenformel C₃₆H₁₈N₄O₄S eingefärbte Prüfkörper zwischen zwei nicht eingefärbten Prüfkörpern mit einem Druck von 4N/cm² bei einer Temperatur von 80°C 24h lang eingeklemmt und anschließend die Migration anhand von Farbstoffrückständen an den zu Beginn farblosen Prüfkörpern visuell bewertet.

Ebenfalls im Rahmen der vorliegenden Erfindung wurde die Temperaturstabilität der mit dem 10,10'-Sulfonyl-bis-12H-phthaloperin-12-on und seiner Isomere eingefärbten und in **Tab. I** beschriebenen Formmassen mittels der 60•40•2 mm³-Platten entsprechend der Norm **DIN EN 12877** geprüft.

| | |
|---|---|
| Komponente A) | Polycarbonat (Makrolon^{®} 2800, Fa. Covestro AG, Deutschland) |
| Komponente B): | Polyamid 6 (Durethan^{®} B30S, Fa. Lanxess Deutschland GmbH, Köln, Deutschland) |
| Komponente C): | Isomere des 10,10'-Sulfonyl-bis-12H-phthaloperin-12-on gemäß Beispiel 1 |

**Tab. 2**

| | | Polymerzusammensetzung 1 | Polymerzusammensetzung 2 |
|---|---|---|---|
| Komponente A) | Massenanteile | 99,8 | - |
| Komponente B) | Massenanteile | - | 99,8 |
| Komponente C) | Massenanteile | 0,2 | 0,2 |
| Migration | | Nicht erkennbar | Nicht erkennbar |
| Temperaturstabilität | °C | 350 | 280 |

Die Ergebnisse in **Tab. 2** zeigen für 10,10'-Sulfonyl-bis-12H-phthaloperin-12-on und seine Isomere keine erkennbare Migration desselben in bzw. aus den Polymerzusammensetzungen 1 und 2 und die Polymerplatten basierend auf 10,10'-Sulfonyl-bis-12H-phthaloperin-12-on und seiner Isomere sowie Polycarbonat und Polyamid 6 zeichneten sich durch eine sehr hohe Temperaturstabilität aus, die bis an die Verarbeitungsgrenzen der untersuchten Polymerzusammensetzungen heranreichte.

## Patentansprüche

1. Sulfonyl-verbrückte Perinone der allgemeinen Formel (I) oder deren isomere Formen 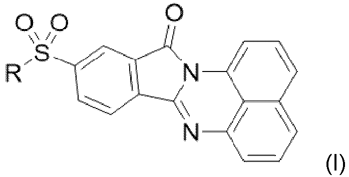 **dadurch gekennzeichnet, dass**
R für C₁-C₆-Alkyl, Aryl, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht.

2. Sulfonyl-verbrückte Perinone gemäß Anspruch 1, **dadurch gekennzeichnet, dass** R für einen Rest 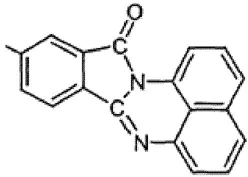 steht.

3. Sulfonyl-verbrückte Perinone gemäß Anspruch 1 oder 2, **dadurch gekennzeichnet, dass** wenigstens einer der Phenylringe wenigstens einen Substituenten der Reihe Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl aufweist.

4. Sulfonyl-verbrückte Perinone gemäß einem oder mehreren der Ansprüche 1 bis 3, **dadurch gekennzeichnet, dass** es sich um 10,10'-Sulfonyl-bis-12H-phthaloperin-12-on handelt.

5. Sulfonyl-verbrückte Perinone gemäß einem oder mehreren der Ansprüche 1 bis 4, **dadurch gekennzeichnet, dass** diese einen Farbabstand ΔE <20 von den L*a*b* Koordinaten zu einer mit "3" beginnenden Farbnummer der RAL-Farbtabelle für die Farbe Rot aufweisen.

6. Sulfonyl-verbrückte Perinone gemäß Anspruch 5, **dadurch gekennzeichnet, dass** diese einen Gelbstich aufweisen wobei a* > 0 und b* ≥ 25 gilt.

7. Verwendung der Sulfonyl-verbrückten Perinone der Formel (I) oder deren isomere Formen 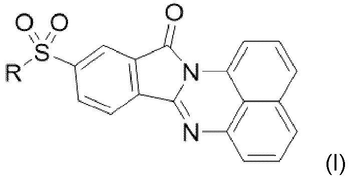 worin
R für C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht, zum Massefärben von Kunststoffen.

8. Verwendung Sulfonyl-verbrückter Perinone gemäß Anspruch 7, **dadurch gekennzeichnet, dass** wenigstens einer der Phenylringe wenigstens einen Substituenten der Reihe Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder Phenyl aufweist.

9. Verwendung Sulfonyl-verbrückter Perinone gemäß Anspruch 7 oder 8, **dadurch gekennzeichnet, dass** darin die Sulfonylbrücke mit denselben Resten zweifach substituiert ist.

10. Verwendung Sulfonyl-verbrückter Perinone gemäß Anspruch 9, **dadurch gekennzeichnet, dass** die Sulfonylbrücke dieselben zwei 12H-Phthaloperin-12-on-Reste miteinander verbrückt.

11. Verwendung Sulfonyl-verbrückter Perinone gemäß Anspruch 10, **dadurch gekennzeichnet, dass** es sich um 10,10'-Sulfonyl-bis-12H-phthaloperin-12-on handelt.

12. Verwendung gemäß einem oder mehreren der Ansprüche 7 bis 11, **dadurch gekennzeichnet, dass** zu färbende Kunststoffe Thermoplaste für die Extrusions, zum Blasformen oder zum Spritzgießen sind, vorzugsweise Vinylpolymere, Polyester, Polyolefine oder Polyamide.

13. Verwendung gemäß Anspruch 12, **dadurch gekennzeichnet, dass** Polyolefine Polyethylen oder Polypropylen bedeuten, Vinylpolymere für Polystyrol, Styrol-Acrylnitril-Copolymere, Styrol-Butadien-Copolymere, Styrol-Butadien-Acrylnitril-Terpolymere, Polymethacrylat oder Polyvinylchlorid steht, Polyester Polyethylenterephthalate, Polybutylenterephthalate, Polycarbonate oder Celluloseester bedeuten und Polyamide für Polyamid 6 oder Polyamid 66 steht.

14. Verwendung gemäß einem oder mehreren der Ansprüche 7 bis 13, **dadurch gekennzeichnet, dass** gleichzeitig das nach DIN 53775-3 zu bestimmende Migrationsverhaltens in diesen Kunststoffen reduziert wird.

15. Verwendung gemäß einem odder mehreren der Ansprüche 7 bis 13, **dadurch gekennzeichnet, dass** gleichzeitig die nach DIN EN 12877 zu bestimmende Temperaturstabilität von Farbstoffen in diesen Kunststoffen gesteigert wird.

16. Verfahren zur Herstellung Sulfonyl-verbrückter Perinone der Formel (I) oder deren isomere Formen 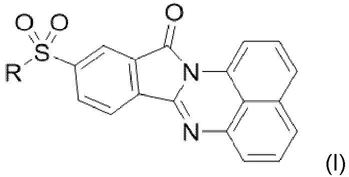 worin
R für C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht,
**dadurch gekennzeichnet, dass** man Sulfonyl-substituierte Isobenzofurandione mit wenigstens einem aromatischen Diamin der Reihe 1,8-Naphthylendiamin, Chlor-1,8-naphthylendiamin, Dichlor-1,8-naphthylendiamin, Methyl-1,8-naphthylendiamin, Dimethyl-1,8-naphthylendiamin, Methoxy-1,8-naphthylendiamin, Ethoxy-1,8-naphthylendiamin, Acetamino-1,8-naphthylen-diamin und 1,8-Diaminoacetnaphthylen umsetzt, vorzugsweise mit 1,8-Naphthylendiamin.

17. Extrusions-, Blasform- oder Spritzgusserzeugnisse basierend auf wenigstens einem Sulfonyl-verbrückten Perinon der allgemeinen Formel (I) oder seiner Isomere 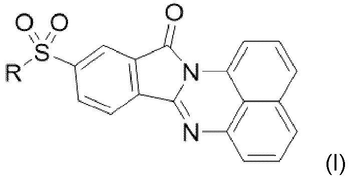 worin
R für C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl oder für einen ankondensierten cycloaliphatischen oder heterocyclischen Rest steht und
wenigstens einem Kunststoff, vorzugsweise wenigstens einem Thermoplasten, besonders bevorzugt wenigstens einem Kunststoff der Reihe Vinylpolymere, Polyester, Polyolefine und Polyamide.
